(19) Europäisches Patentamt
European Patent Office
Office européen des brevets

(11) EP 0 727 495 A2

(12) EUROPEAN PATENT APPLICATION

(43) Date of publication:
**21.08.1996 Bulletin 1996/34**

(51) Int. Cl.[6]: **C12Q 1/48**, C12Q 1/54

(21) Application number: **96102190.4**

(22) Date of filing: **14.02.1996**

(84) Designated Contracting States:
**DE FR GB**

(30) Priority: **17.02.1995 JP 53404/95**
**17.02.1995 JP 53405/95**
**14.11.1995 JP 319502/95**

(71) Applicant: **KUREHA CHEMICAL INDUSTRY CO., LTD.**
**Chuo-ku Tokyo (JP)**

(72) Inventors:
- **Takahashi, Eisaku**
**Tokyo (JP)**
- **Fujimoto, Satoshi**
**Iwaki-shi, Fukushima (JP)**
- **Kase, Toshiya**
**Warabi-shi, Saitama (JP)**

(74) Representative: **Boeters, Hans Dietrich, Dr. et al**
**Patentanwälte Boeters & Bauer,**
**Bereiteranger 15**
**81541 München (DE)**

# (54) Quantitation of inorganic phosphate and trehalose

(57) The present invention relates to quantitation of trehalose or inorganic phosphate and reagent used therein. More specifically, the present invention provides a method of quantitating trehalose or inorganic phosphate in a trehalose containing sample or in an inorganic phosphate containing sample by determining the amount of α-D-glucose 1-phosphate or that of D-glucose produced in a reaction of trehalose with inorganic phosphate catalyzed by trehalose phosphorylase.

Fig. 1

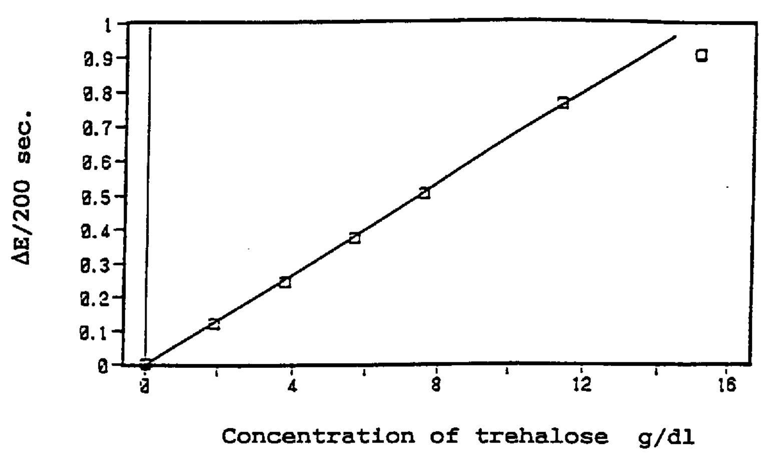


EP 0 727 495 A2

## Description

### Field of the Invention

The present invention relates to quantitation of inorganic phosphate (including acid form thereof) or trehalose using trehalose phosphorylase and reagents for quantitaion thereof. More specifically, the present invention relates to a method of quantitating inorganic phosphate or trehalose in a sample by determining the amount of α-D-glucose 1-phosphate (including acid form thereof) or D-glucose which are produced in a reaction of trehalose with inorganic phosphate in the presence of trehalose phosphorylase and reagents for quantitation thereof.

### Background of the Invention

Trehalose has characteristics such as a protective action for protein dryness, a protective action for protein freeze drying, low sweetness, non-reducing property, wet reservative property etc. and utility thereof are suggested for pharmaceutical (Japanese published unexamined patent application 72883 (1994)), foods (Japanese published unexamined patent application 253722 (1994)), cosmetics (Japanese published unexamined patent application 40815 (1994)) and so on. In theses fields, a method of quantitaing trehalose in a small amount of sample is required. In a method of quantitating various organic compounds, many enzyme reactions have been used so far. However, a practical method of quantitating trehalose using enzymic reaction is not known yet.
On the other hand, phosphoric acid is an essential substance for carbohydrate metabolism as various phosphoric acid esters and for energy metabolism as high energy phosphoric compounds in a living body. And in the case of dysfunction of renal excretion and/or reabsorption of phosphate, hyperphosphatemia and/or hypophosphatemia etc. are observed clinically. For diagnosis and/or treatment thereof, it is important to quantitate the amount of inorganic phosphate. Further, almost all foods contain phosphate and it is also important to quantitate phosphate for quality control in the case of preparing phosphate-limited foods in hospital, processing and preparing general foods. As a method of quantitating inorganic phosphate, Molybdenum-blue method is well known up to now. However, this method is apt to be affected very much by a reducing substance and, moreover, burdens the environment with a harmful substance such as a strong acid. To solve the above-mentioned problems, a method using sucrose phosphorylase (Japanese published unexamined patent application 49100 (1988), Japanese published examined patent application 53078 (1994) and a method using pyruvic acid oxidase were described. In the above former patent, a method of quantitating fructose and α-D-glucose 1-phosphate produced in an enzymic reaction was described, wherein disturbance by glucose,fructose and/or xyeulose (intermediate thereof in a living body) in a sample and substrate specificity of sucrose phosphorylase must be strongly considered. (Denpun Kagaku, vol.40, No.1, p1-5 (1993), J. Bacteriol., 68, 381-388 (1954), Oyo Toshitu Kagaku vol. 41, No.2, p165-172 (1994)).
The present inventors found that a novel trehalose phosphorylase which catalyzes a reaction of trehalose with inorganic phosphate to produce α-D-glucose 1-phosphate and D-glucose and filed a Japanese patent application No. 76461 (1994)). By further investigation on this trehalose phosphorylase, it was found that the above reaction proceeded quantitatively and a reaction of produced α-D-glucose 1-phosphate in the presence of an electron acceptor by phosophglucomutase and D-glucose-6-phosphate dehydrogenase quantitatively converted the electron acceptor into a reduced form of electron acceptor and, in addition, the electron acceptor was quantitatively converted into a reduced form of electron acceptor by a reaction of α-D-glucose 1-phosphate with phosphoglucomutase D-glucose-6-phosphate dehydrogenase and 6-phosphogluconate dehydrogenase. In addition, it was also found that a reaction of D-glucose in the presence of an electron acceptor with mutarotase, glucose oxidase and peroxidase quantitatively converted the electron donor into an oxidized form of electron donor. The present invention was accomplished from these observations.
Accordingly, as an object of the present invention, the present inventors have investigated in order to provide a method of quantitating specifically trehalose or inorganic phosphate in a small amount of sample and a quantitating agent thereof suitable for a kit for a biochemical autoanalyzer, a kit for manual analysis,a biosensor, a testing paper and reached the present invention. As a biochemical autoanalyzer, a flow type, a discrete type(a reactor type, a packed type or a centrifuge type), or a film type can be used.

### Summary of the Invention

Accordingly, an object of the present invention is to provide a method of quantitating trehalose or inorganic phosphate comprising the following steps:
A sample containing trehalose or inorganic phosphate is allowed to react with inorganic phosphate or trehalose in the presence of trehalose phosphorylase to produce α-D-glucose 1-phosphate and D-glucose; The produced α-D-glucose 1-phosphate or D-glucose is quantitated.
Another object of the present invention is to provide a reagent for quantitating trehalose or inorganic phosphate comprising trehalose phosphorylase which catalyzes a reaction of trehalose with inorganic phosphate to produce α-D-glucose 1-phosphate and D-glucose, phosphoglucomutase, D-glucose-6-phosphatedehydrogenase, inorganic phosphate or trehalose, D-glucose 1,6-diphosphate and an electron acceptor.

Another object of the present invention is to provide a reagent for quantitating trehalose or inorganic phosphate comprising trehalose phosphorylase which catalyzes a reaction of trehalose with inorganic phosphate to produce α-D-glucose 1-phosphate and D-glucose, phosphoglucomutase, D-glucose-6-phosphatedehydrogenase, 6-phosphogluconate dehydrogenase, inorganic phosphate or trehalose, D-glucose 1,6-diphosphate and an electron acceptor.

Further, another object of the present invention is to provide a method of quantitating trehalose or inorganic phosphate comprising the steps of reacting trehalose with inorganic acid in the presence of trehalose phosphorylase, reacting produced D-glucose with mutarotase, glucose oxidase and peroxidase in the presence of an electron acceptor and determining the amount of oxidized form of electron acceptor converted from the electron acceptor. The present invention relates to qunantitaion of trehalose or inorganic acid characterized by determining the amount of α-D-glucose 1-phosphate or D-glucose produced in a reaction of trehalose with inorganic phosphate catalyzed by trehalose phosphorylase with addition of excessive inorganic phosphate in the case of a trehalose containing sample and with addition of trehalose in the case of an inorganic phosphate containing sample.

In addition, the present invention relates to a reagent for quantitating trehalose or inorganic phosphate comprising trehalose phosphorylase which catalyzes a reaction of trehalose with inorganic phosphate to produce α-D-glucose 1-phosphate and D-glucose, phosphoglucomutase, D-glucose-6-phosphatedehydrogenase, inorganic phosphate or trehalose, D-glucose 1,6-diphosphate and an electron acceptor.

Further, the present invention relates to a reagent for quantitating trehalose or inorganic phosphate comprising trehalose phosphorylase, mutarotase, glucose oxidase, peroxidase and an electron donor.

Further, another object of the present invention is to provide a method of quantitating trehalose or inorganic phosphate comprising the steps of reacting trehalose with inorganic phosphate in the presence of trehalose phosphorylase, reacting produced D-glucose with mutarotase, glucose oxidase and peroxidase in the presence of an electron donor and determining the amount of oxidized form of electron donor converted from the electron donor.

In the present invention, determination of α-D-glucose 1-phosphate can be carried out by a reaction thereof catalyzed by an enzyme and determining the amount of reduced form of electron acceptor produced therein. In addition, determination of D-glucose can be carried out by enzymic reaction followed by determining the amount of oxidized form of electron donor produced therein.

## Brief Description of Drawings

Figure 1 shows relationship between the concentration of trehalose and ΔE/200 sec. at 340 nm.

Figure 2 shows relationship between the concentration of trehalose and ΔE/200 sec. at 340 nm.

Figure 3 shows relationship between the concentration of trehalose and absorbance at 505 nm.

Figure 4 shows correlation between the results of the determination of inorganic phosphate by the method of the present invention and those of Molyodenum-blue method.

## Detailed Description of the Invention and Preferred Embodiment

Trehalose phosphorylase used in the present invention is an enzyme which catalyzes not only a reaction of trehalose with inorganic phosphate to produce α-D-glucose 1-phosphate and D-glucose, but also the reversed reaction to produce trehalose from D-glucose 1-phosphate and D-glucose and can be produced by fungi such as Grifora frondosa, Pleurotus ostreatus, Lyophyllum ulmarium, Lentinus edodes and so on.

Trehalose phosphorylase catalyzes a reaction of trehalose with inorganic phosphate to produce α-D-glucose 1-phosphate and D-glucose shown in reaction formula 1 described below.

[Reaction formula 1]

$$\text{Trehalose + inorganic phosphate} \xrightarrow{\text{Trehalose phosphorylase}} \alpha\text{-D-glucose 1-phosphate + D-glucose}$$

D-glucose 6-phosphate is produced by a reaction of α-D-glucose 1-phosphate produced in reaction formula 1 with D-glucose 1,6-diphosphate catalyzed by phosphoglucomutase (EC 2.7.5.1, Enzyme handbook, Asakura-shoten, 1982) as described in reaction formula 2. Produced D-glucose 6-phosphate is converted into 6-phospho-D-gluconate by catalysis of D-glucose-6-phosphate dehydrogenase (EC 1.1.1.49) as described in reaction formula 3. In the reaction of D-glucose 6-phosphate to 6-phospho-D-gluconic acid, an electron acceptor present therein is converted into a reduced form of electron acceptor. In reaction formula 3, as an electron acceptor, nicotinamide adenine dinucleotide (NAD), nicotinamide adenine dinucleotide phosphate (NADP), nicotinamide hypoxanthine dinucleotide (deaminoNAD), nicotinamide hypoxanthine dinucleotide phosphate (deaminoNADP), acetylpyridine adenine dinucleotide (acetylNAD), acetylpyridine adenine dinucleotide phosphate (acetylNADP), acetylpyridine hypoxanthine dinucleotide or acetylpyridine hypoxanthine dinucleotide phosphate (hereinafter abbreviated as NAD(P)s generically) can be used and determination of trehalose or inorganic phosphate in a sample can be carried out by determining the amount of conversion thereof into reduced forms of NAD(P)s. In addition, by cyclising thioNAD(P)s such as thionicotinamide dinucleotide phosphate (thioNADP), thionicotinamide hypoxanthine dinucleotide phosphate, thionicotinamide hypoxanthine dinucleotide and so on, sensitivity of determination can be amplified. (Japanese published unexamined patent application 335898 (1992), Japanese published unexamined patent application 224498 (1991))

[Reaction formula 2]

$\alpha$-D-glucose 1-phosphate + D-glucose 1,6-diphosphate $\xrightarrow{\text{phosphoglucomutase}}$ D-glucose 6-phosphate + D-glucose 1,6-diphosphate

[Reaction formula 3]

$$\text{D-glucose 6-phosphate} \xrightarrow[\text{NAD(P)s} \;\rightarrow\; \text{NAD(P)Hs}]{\text{D-glucose-6-phosphate dehydrogenase}} \text{6-phospho-D-gluconate}$$

D-glucose 1,6-diphosphate used in reaction formula 2 is necessary for activation of phosphoglucomutase.Inorganic phosphate in determination of trehalose is preferably used as a form of buffer solution. The present invention is preferably carried out by the initial reaction rate method. Especially in determining inorganic phosphate, the initial reaction rate method in particularly preferable so that bilirubin and hemoglobin contaminated in a sample serum or urine will not affect on the analytical results. The end point method wherein trehalose is consumed in the case of determination of trehalose, on the other hand, inorganic phosphate is consumed in the case of determining inorganic phosphate can be used.

On determining trehalose or inorganic phosphate by analyzing the amount of $\alpha$-D-glucose 1-phosphate produced in a reaction of trehalose with inorganic phosphate catalyzed by trehalose phosphorylase, a sample can be mixed with a solution comprising trehalose phosphorylase, phosphoglucomutase D-glucose-6-phosphate dehydrogenase and D-glucose 1,6-diphosphate and a produced reduced form of electron acceptor can be analyzed.

On determining trehalose, the concentration of inorganic phosphate in reagents may be used more than 5 times, preferably 10 times, comparing to the expected concentration of trehalose in a sample to be analyzed and, on determination of inorganic phosphate, the concentration of trehalose may be used more than 50 times, preferably 100 times, comparing to the expected concentration of inorganic phosphate in a sample to be analyzed.

Accordingly, the present invention provides an agent for quantitating trehalose or inorganic phosphate comprising trehalose phosphorylase which catalyzes a reaction of trehalose with inorganic phosphate to produce $\alpha$-D-glucose 1-phosphate and D-glucose, phosphoglucomutase, D-glucose-6-phosphatedehydrogenase, D-glucose 1,6-diphosphate and an electron acceptor.

By combining 6-phosphogluconate dehydrogenase (EC 1.1.1.43 or EC1.1.1.44) with the above quantitaing reagents of the present invention, 6-phosphogluconate (including acid form thereof) produced in reaction formula 3 reacts further to produce a reduced form of electron acceptor as described in reaction formula 4 or 5.

Accordingly, one mole of trehalose or inorganic phosphate can produce 2 moles of reduced form of electron acceptor, which can decrease the sample amount and is preferable. Normal serum level of inorganic phosphate in an adult is as low as 3.5-4.5 mg/100 ml. Therefore, a method with high sensitivity for quantitating inorganic phosphate is required and the present invention can be used preferably.

[Reaction formula 4]

6-phospho-D-gluconate —(6-phosphogluconate dehydrogenase; NAD(P)s → NAD(P)Hs)→ D-ribrose 5-phosphate + $CO_2$

[Reaction formula 5]

6-phospho-D-gluconate —(6-phosphogluconate dehydrogenase; NAD(P)s → NAD(P)Hs)→ 2-keto-6-phospho-D-gluconate

Another method of the present invention is a method of determining D-glucose produced in a reaction of trehalose with inorganic phosphate catalyzed by trehalose phosphorylase. That is, reactions of D-glucose with mutarotase (EC5.1.3.3), glucose oxidase (EC1.1.3.4), peroxidase (EC1.11.1.7) and an electron donor convert the electron donor into an oxidized form of electron donor.

[Reaction formula 6]

$$\alpha\text{-D-glucose} \xrightarrow{\text{mutarotase}} \beta\text{-D-glucose}$$

[Reaction formula 7]

$$\beta\text{-D-glucose} \xrightarrow{\text{glucose oxidase}} \text{gluconate + hydrogen peroxide}$$

[Reaction formula 8]

$$\text{hydrogen peroxide + electron donor} \xrightarrow{\text{peroxidase}} \text{oxidized form of electron donor} + 2\ H_2O$$

As an electron donor, for example, 4-aminoantipyrin and phenol or 4-aminoantipyrin and 3,5-dimethoxyanilinopropane sulfonic acid which is quantitatively converted into red or blue quinone chromophore can be used. Trehalose or inorganic phosphate can be quantitated by analyzing absorption of this chromophore which is an oxidized form of electron donor produced.

Accordingly, the present invention provides reagents for quantitating trehalose or inorganic phosphate comprising trehalose phosphorylase which catalyzes a reaction of trehalose with inorganic phosphate to produce α-D-glucose 1-phosphate and D-glucose, mutarotase, glucose oxidase, peroxidase and an electron donor.

To practice the method of the present invention, the amount of trehalose phosphorylase to be used is 0.01-2000 units/ml, preferably 0.05-500 units/ml, more preferably 0.1-20 units/ml. The concentration of inorganic phosphate in quantitating trehalose or the concentration of trehalose in quantitating inorganic phosphate is 10-500 mM, preferably 50-200 mM.

As a buffer solution used in the present invention, a suitable buffer solution may be selected by considering enzymic activity. For example, to quantitate trehalose, good buffers such as phosphate buffer solution, Tris buffer solution like Tris-maleic acid, Tris-acetic acid, Tris-hydrochloride etc., HEPES (N-2-Hydroxyethyl piperazine-N'-2-ethanesulfonic acid) or MES(2-[(N-Morpholino) ethanesulfonic acid, monohydrate]) can be used. Particularly,phosphate buffer solution can be preferably used. Alternately, as a buffer solution for quantitating inorganic phosphate, a buffer solution not containing inorganic phosphate can be used and, for example, buffers described above except phosphate buffer can be used.

The salt concentration of a buffer solution is not limited as far as enzymic activity is not inhibited and, for example, 20-200 mM is preferable. The pH is preferably 5.5-8.5.

The concentration of an electron acceptor in a reaction where α-D-glucose 1-phosphate produced by the action on trehalose phosphorylase (reaction formula 2, 3, 4 and 5) is determined, is 1-50 mM, preferably 1-5 mM. And the amount of phosphoglucomutase, D-glucose-6-phosphate dehydrogenase and 6-phosphogluconic acid dehydrogenase used therein is 0.01-1000 units/ml, preferably 0.05-100 units/ml, more preferably 0.1-10 units/ml. For activating phosphoglucomutase, the concentration of D-glucose 1,6-diphosphate (including acid form thereof) can be 0.001-2 mM, preferably 0.01-0.2 mM and the concentration of $Mg^{2+}$ can be 0.1-100 mM, preferably 1-10 mM. Among D-glucose-6-phosphate dehydrogenases and 6-phosphogluconic acid dehydrogenases, there are not only HAD dependent enzymes but also NADP dependent enzymes depending on their origin. Therefore, an appropriate coenzyme can be chosen according to enzymic properties.

On the other hand, in a reaction where D-glucose produced by the action of trehalose phosphorylase is determined (reaction formula 6,7 and 8), the amount of trehalose phosphorylase used therein can be 0.01-2000 units/ml, preferably 0.05-500 units/ml, more preferably 0.1-20 units/ml. The amount of mutarotase used therein can be 0.01-2000 units/ml, preferably 0.05-500 units/ml, more preferably 0.1-20 units/ml. The amount of glucose oxidase used therein can be 0.01-10000 units/ml, preferably 0.05-1000 units/ml, more preferably 0.1-100 units/ml. The amount of peroxidase used therein can be 0.001-2000 units/ml, preferably 0.005-500 units/ml, more preferably 0.01-20 units/ml.

The concentration of an electron donor such as 4-aminoantipyrin and phenol or 3,5-dimethoxyanilinopropane sulfonic acid can be selected sufficiently enough for produced hydroperoxide to be quantitatively converted for color development.

Further, it is preferable for mutarotase to be present in reagents because it is an enzyme converting α-glucose into β-glucose immediately after production thereof by the action of trehalose phosphorylase.

To practice the present invention, it can be selected as to whether α-D-glucose 1-phosphate or D-glucose is analyzed according to a composition of a sample. For example, determination of α-D-glucose 1-phosphate is preferable for quantitating trehalose or inorganic phosphate in serum containing D-glucose. The method of the present invention for quantitating α-D-glucose 1-phosphste is not inhibited by the presence of D-glucose. Alternately, in the case of analyzing D-glucose in a sample containing D-glucose, quantitation of trehalose or inorganic phosphate can be carried out after D-glucose in the sample is removed by enzymic treatment such as glucose oxidase in advance.

In addition, substrate specificity of trehalose phosphorylase for monosaccharide or sugar phosphate were investigated and it was found that monosaccharide except D-glucose, that is, L-glucose, D-galactose, D-mannose, D-xylose, D-fructose, D-sorbitol, D-mannitol, D-fucose, D-glucuronic acid, N-acetyl-D-glucosamine, N-acetylneuraminic acid did not react with the enzyme, either of sugar phosphate except α-glucose 1-phosphate, that is, β-glucose 1-phosphate, α-galactose 1-phosphate, α-mannose 1-phosphate, α-xylose 1-phosphate did not react with the enzyme. The above reaction was not inhibited by the presence of monosaccharide or sugar phosphate described above. Trehalose and inorganic phosphate can specifically be quantitated in a small amount of sample according to the method of the present invention, which can be carried out using an autoanalyzer. The present invention will be explained in more detail by exemplifying examples as below. However, the scope of the present invention is not limited by those examples. Analysis of absorbance in the following examples was carried out by using a spectrophotometer of Shimazu UV-2200.

[Example 1]

| Regents composition | |
|---|---|
| Phosphate buffer solution | 200 mM, pH 6.5 |
| NADP | 3.0 mM |
| Magnesium chloride | 1.3 mM |
| D-Glucose 1,6-diphosphate | 0.067 mM |
| Phosphoglucomutase | 3.0 u./ml |
| D-Glucose-6-phosphate dehydrogenase | 3.5 u./ml |
| Trehalose phosphorylase | 0.5 u./ml |

Reagents of the above composition (1.95 ml) was warmed in advance and 50 µl of a trehalose solution which was prepared in various concentrations was added thereto. Analytical results of ΔE/200 sec at 340 nm( difference between absorbance at 340 nm at immediately after the initiation of a reaction and that at 200 seconds after the initiation

thereof.) are shown in figure 1.
It is clear from figure 1 that good linearlity was obtained below 12 g/dl (300 mM) of trehalose.

[Example 2]

| Reagents composition | |
|---|---|
| Phosphate buffer solution | 200 mM, pH 6.5 |
| NADP | 3.0 mM |
| Magnesium chloride | 1.3 mM |
| D-Glucose 1,6-diphosphate | 0.067 mM |
| Phosphoglucomutase | 3.0 mM |
| D-Glucose-6-phosphate dehydrogenase | 3.5 u./ml |
| 6-Phosphpgluconate dehydrogenase | 3.0 u./ml |
| Trehalose phosphorylase | 0.5 u./ml |

Reagents of the above composition(1.95 ml) was warmed in advance and 50 µl of samples of various concentration as described in the example 1 were added thereto and the analytical results of ΔE/200 sec. at 340 mare shown in figure 2. It is clear from figure 2 that good lineality was obtained below 8 g/dl (230 mM) of trehalose.

[Example 3]

| Reagents composition | |
|---|---|
| Phosphate buffer solution | 100 mM, pH 6.5 |
| 4-Aminoantipyrin | 1.0 mM |
| Phenol | 5.3 mM |
| Mutarotase | 10 u./ml |
| Glucose oxidase | 10 u./ml |
| Peroxidase | 1.0 u./ml |
| Trehalose phosphorylase | 1.0 u./ml |

Reagents of the above composition(1.95 ml)was warmed in advance and 50 µl of sample of various concentration as described in the example 1 was added thereto. After a reaction was carried out at 30°C for 30 minutes,absorbance at 505 nm was analyzed. The results are shown in figure 3. It is clear from figure 3 that good linearlity was obtained below 14 g/dl (400 mM) of trehalose.

[Example 4]

Two hundreds ml of purified water was added to 100 g of commercially available mushroom (maitake or fruitbody of Grifola frondosa) and undissolved substance was removed by centrifugation to give 248 ml of supernatant. The concentration of trehalose in the supernatant was analyzed in the same manner as example 1 and the concentration of trehalose in the sample was 15.6 mM, which means that 100 g of the mushroom contained 1.5 g of trehalose( as trehalose 2 hydrate). Further, the concentration of trehalose of the sample was 15.5 mM by analysis of HPLC under the conditions described below.

Method of analyzing trehalose by HPLC:

| | |
|---|---|
| Column: | Polyamine column (YMC Pack™ PolyamineII 4.6 mm∅ x 250 mm, YMC, Japan) |
| Elution solution: | Acetonitrile/water = 70/30, flowing rate 1 ml/min. |
| Column temperature: | 35°C |
| Detector: | Differential refractometer (cell temperature: 35°C) |

[Example 5]

| Reagents composition | |
|---|---|
| MES buffer solution | 200 mM, pH 6.5 |
| Trehalose | 100 mM |
| $NADP^+$ | 3.3 mM |
| Magnesium chloride | 1.3 mM |
| D-glucose 1,6-diphosphate | 0.067 mM |
| Phosphoglucomutase | 0.5 u./ml |
| D-Glucose-6-phosphate dehydrogenase | 0.5 u./ml |
| Trehalose phosphorylase | 0.5 u./ml |

To reagents of the above composition(2.95 ml), 50 µl of sample was added and left at 30°C for 30 minutes. Absorbance at 340 nm thereof was analyzed and good lineality through the origin was obtained below 50 mg/dl (16 mM) inorganic phosphate. Further, glucose was added to the above reagent composition so that the final concentration of glucose became 200 mg/dl (11 mM), followed by analysis of concentration of inorganic phosphate thereof. Comparing to the results without addition of glucose, the analytical value with addition of glucose was within the region of 100 ± 5 %. It was found that quantitation of inorganic phosphate was not inhibited by the presence of glucose.
Correlation between the present method and conventional method (molybdenum-blue method) is shown in figure 4. It was clear from figure 4 that good correlation was obtained (r=0.998, y=1.46x, x:molyodenum-blue method, y:the present method).

[Example 6]

Serum inorganic phosphate from 3 normal men fasted overnight was determined in the same manner as example 1 (4.1, 3.8 and 3.6 mg/dl respectively). To the same sera, inorganic phosphate was purposely added so as to increase 1 mg/dl more in each serum. The analytical results were 5.0, 4.8 and 4.7 mg/dl.

**Claims**

**1.** A method of quantitating trehalose or inorganic phosphate characterized by determining by the following steps:

i. adding inorganic phosphate or trehalose in a trehalose containing sample or in an inorganic phosphate containing sample;
ii. reacting trehalose with inorganic phosphate in the presence of trehalose phosphorylase to produce α-D-glucose 1-phosphate and D-glucose; and
iii. determining the amount of produced α-D-glucose 1-phosphate or that of produced D-glucose.

**2.** The method according to claim 1, wherein a reduced form of electron acceptor is produced by a reaction of said produced α-D-glucose 1-phosphate with phosphoglucomutase and D-glucose-6-phosphate dehydrogenase in the presence of an electron acceptor and the amount thereof is determined.

3. The method according to claim 1, wherein a reduced form of electron acceptor is produced by a reaction of said produced α-D-glucose 1-phosphate with phosphoglucomutase D-glucose-6-phosphate dehydrogenase and 6-phosphogluconate dehydrogenase in the presence of an electron acceptor and the amount thereof is determined.

4. The method according to claim 2 or 3, wherein an electron acceptor is selected from the group comprising nicotinamide adenine dinucleotide (NAD), nicotinamide adenine dinucleotide phosphate (NADP), nicotinamide hypoxanthine dinucleotide (deaminoNAD), nicotinamide hypoxanthine dinucleotide phosphate (deaminoNADP), acetylpyridine adenine dinucleotide (acetylNAD), acetylpyridine adenine dinucleotide phosphate (acetylNADP), acetylpyridine hypoxanthine dinucleotide, or acetylpyridine hypoxanthine dinucleotide phosphate (NAD(P)s).

5. The method according to any one of claims 2 to 4, wherein a cycling reaction of one selected from NAD(P)s with a coenzyme selected from the group comprising thionicotinamide adenine dinucleotide, thionicotinamide adenine dinucleotide phosphate, thionicotinamide hypoxanthine dinucleotide and thionicotinamide hypoxanthine dinucleotide phosphate is formed.

6. The method according to claim 1, wherein an oxidized form of electron donor is produced by a reaction of said produced D-glucose with mutarotase, glucose oxidase and peroxidase in the presence of an electron donor and the amount thereof is determined.

7. Reagents for quantitaing trehalose or inorganic phosphate comprising trehalose phosphorylase which catalyzes a reaction of trehalose with inorganic phosphate to produce α-D-glucose 1 phosphate and D-glucose, phosphoglucomutase, D-glucose-6-phosphate dehydrogenase, inorganic phosphate or trehalose , D-glucose 1,6-diphosphate and an electron acceptor.

8. Reagents for quantitating trehalose or inorganic phosphate comprising trehalose phosphorylase which catalyzes a reaction of trehalose with in organic phosphate to produce α-D-glucose 1-phosphate and D-glucose, phosphoglucomutase, D-glucose-6-phosphate dehydrogenase, 6-phosphogluconate dehydrogenase, inorganic phosphate or trehalose, D-glucose 1,6-diphosphate and an electron acceptor.

9. The reagents according to claim 7 or 8, wherein an electron acceptor is selected from NAD(P)s.

10. The reagents according to any one of claims 7 to 9, wherein a cycling reaction of one selected from NAD(P)s with a coenzyme selected from the group comprising thionicotinamide adenine dinucleotide, thio-nicotinamide adenine dinucleotide phosphate, thionicotinamide hypoxanthine dinucleotide and thionicotinamide hypoxanthine dinucleotide phosphate is formed.

11. Reagents for quantitating trehalose or inorganic phosphate comprising trehalose phosphorylase which catalyzes a reaction of trehalose with inorganic phosphate to produce D-glucose 1-phosphate and D-glucose, mutarotase, glucose oxidase, peroxidase, inorganic phosphate or trehalose and an electron donor.

Fig. 1

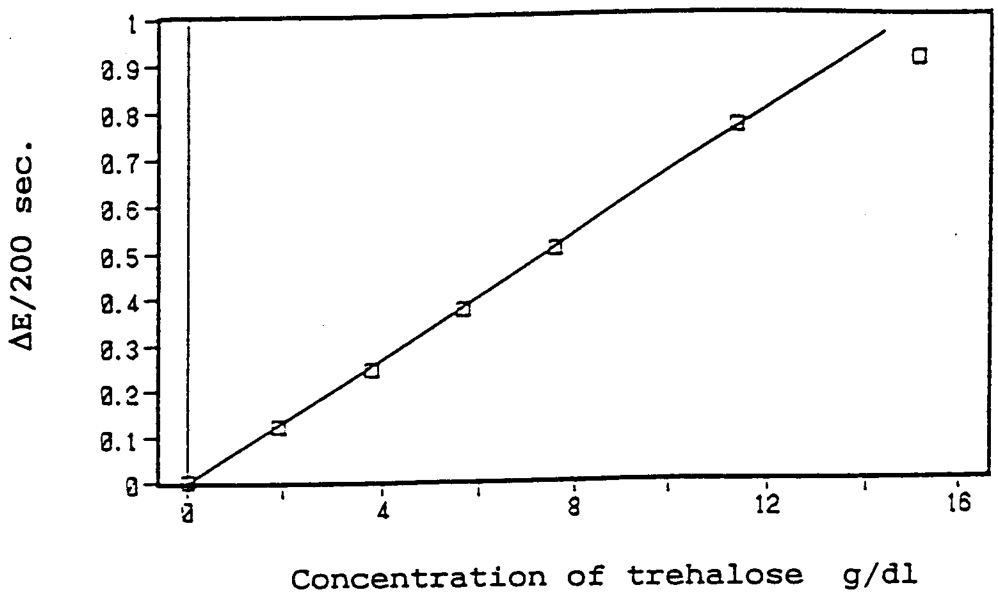
1
0.9
0.8
0.7
0.6
0.5
0.4
0.3
0.2
0.1
0
ΔE/200 sec.
0
4
8
12
16
Concentration of trehalose g/dl

Fig. 2

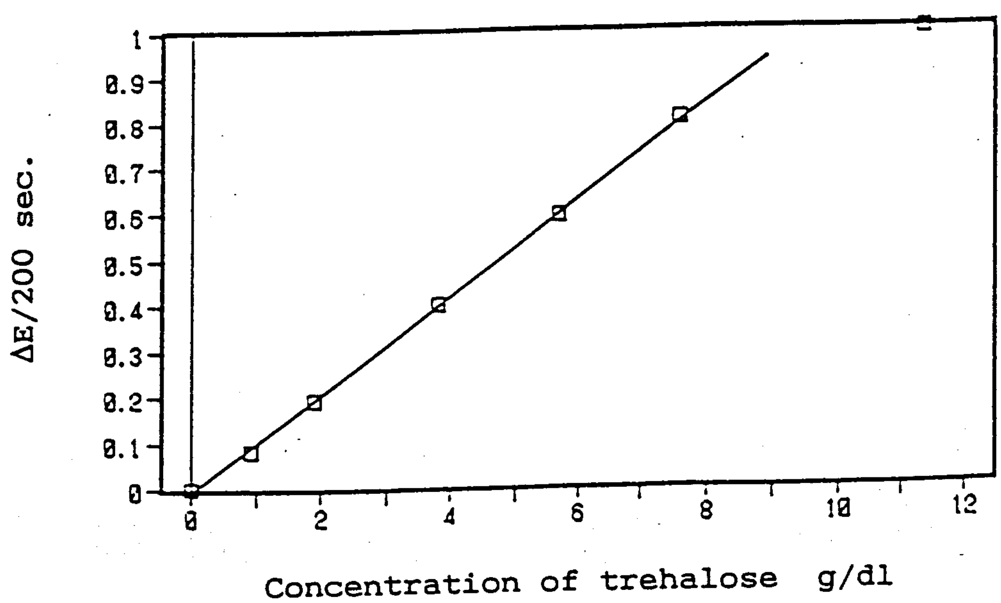
1
0.9
0.8
0.7
0.6
0.5
0.4
0.3
0.2
0.1
0
ΔE/200 sec.
0
2
4
6
8
10
12
Concentration of trehalose g/dl

Fig. 3

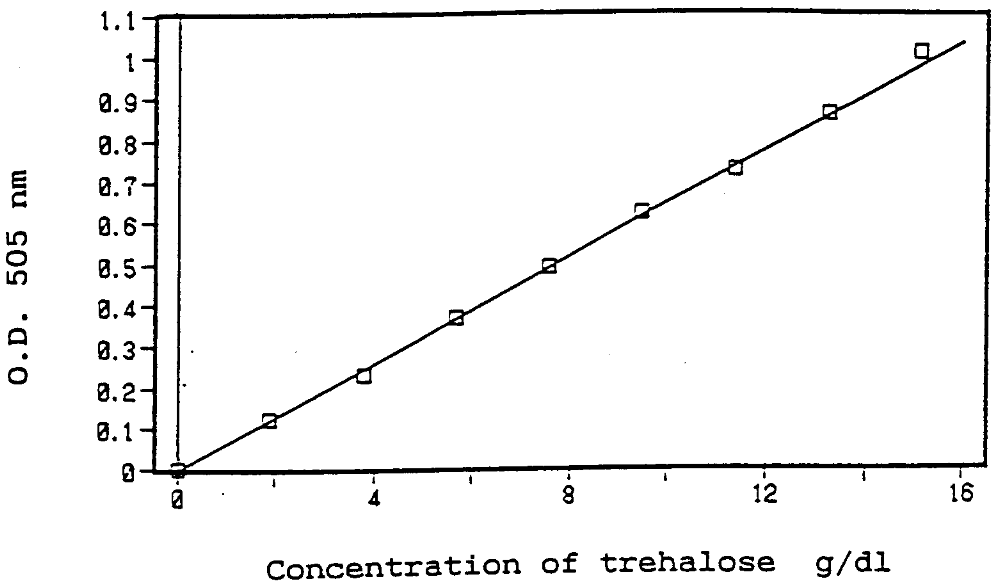
1.1
1
0.9
0.8
0.7
0.6
0.5
0.4
0.3
0.2
0.1
0
O.D. 505 nm
0
4
8
12
16
Concentration of trehalose g/dl

Fig. 4

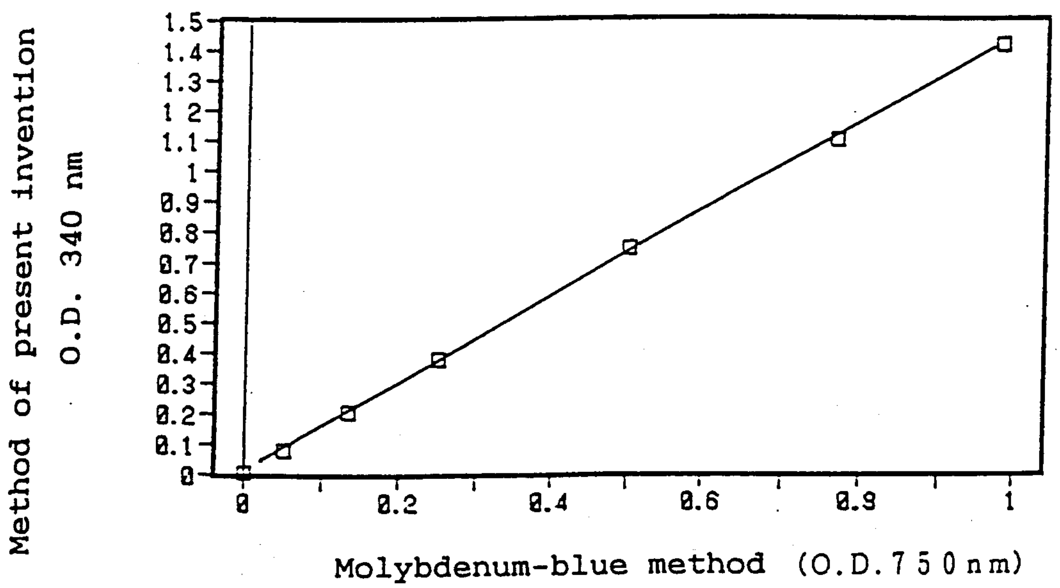
1.5
1.4
1.3
1.2
1.1
1
0.9
0.8
0.7
0.6
0.5
0.4
0.3
0.2
0.1
0
Method of present invention
O.D. 340 nm
0
0.2
0.4
0.6
0.8
1
Molybdenum-blue method (O.D.750nm)